# EUROPEAN PATENT APPLICATION

(11) **EP 4 375 370 A1**
(43) Date of publication of application: **29.05.2024**
(21) Application number: 22306741.4
(22) Date of filing: 25.11.2022
(51) Int. Cl.: C12N 9/14, C12N 15/62

(54) **FULLY FUNCTIONAL TAGGED RAD51 PROTEIN AND USES THEREOF**

(71) Applicant: Institut Curie, 75005 Paris (FR); Commissariat à l'Énergie Atomique et aux Énergies Alternatives, 75015 Paris (FR); Centre National de la Recherche Scientifique, 75016 Paris (FR)
(72) Inventor: The designation of the inventor has not yet been filed
(74) Representative: A.P.I. Conseil

(57) **Abstract**

Invention belongs to the field of molecular biology. Invention relates to a fully functional tagged Rad51 protein which constitutes a valuable tool for studying the homologous recombination pathway and alternative lengthening of telomeres. More particularly the invention relates to a fully functional tagged Rad51 protein comprising a tag between residues 54 and 55 of Pad 51 protein of SEQ ID NO:1 or between corresponding residues of any budding yeast ortholog thereof. Accordingly, the invention relates also to methods of screening candidate compounds capable of interfering with such mechanisms and to method of screening drugs useful in cancer treatment.

## Description

### FIELD OF THE INVENTION

Invention belongs to the field of molecular biology. More specifically invention belongs to the fields of biotechnology tools and drug discovery. Invention relates to a fully functional tagged Rad51 protein which constitutes a valuable tool for studying the homologous recombination pathway and alternative lengthening of telomeres. Accordingly, the invention also relates to methods of screening candidate compounds capable of interfering with such mechanisms and to method of screening drugs useful in cancer treatment.

### BACKGROUND OF THE INVENTION

Rad51 is a eukaryotic ortholog of the bacterial RecA recombinase which plays a key role in the repair of double strand DNA breaks, a process termed homologous recombination (HR), occurring during both the normal cell cycle and repair of DNA damage. Rad51 is also a key player in the alternative lengthening of telomeres (ALT) recombination pathway.

Excess cellular Rad51 occurs in many cancers and leads to resistance to DNA-damaging radiation and chemotherapy in addition to genomic instability. Also, one critical mechanism for Rad51 control seems to involve its interaction with the breast cancer susceptibility protein BRCA2, whose inactivation predisposes to breast, ovarian and other cancers. Rad51 is also central to the ALT recombination pathway that has been shown to be involved in the maintenance of telomere length in numerous cancer cells.

Accordingly, in addition to treatment resistance mechanisms, Rad51 is an interesting target candidate for cancer prognosis and therapy.

Cancer is the second leading cause of death globally and is responsible for an estimated 9.6 million deaths in 2018. Globally, about 1 in 6 deaths is due to cancer (World Health Organization, <https://www.who.int>). In Europe, cancer incidence is continuously growing and increased by around 50 percent from 2.1 million to 3.1 million cases between 1995 and 2018 (Hofmacher et al., 2019).

Accordingly, the economic impact of cancer is significant and is continuously increasing. Direct costs of cancer doubled from €52 billion to €103 billion in Europe between 1995 and 2018 (Hofmacher et al., 2019), to which should be added significant economic burden of productivity losses due to premature deaths.

Expression of C- or N- terminal GFP-tagged Rad51 constructs in plants (Kobayashi et al. Genes Genet Syst, 2014; Olivier et al. Nuc Acid Res, 2018), yeast (Cloud et al. Science, 2012; Waterman et al. bioRxiv, 2018), mouse (Reuter et al. JCB, 2014) and human (Yu et al. Mol Cell, 2003; Robertson et al., 2009) cells have been employed to trace the dynamics of Rad51 compartmentalization in live cells upon DNA damage and in conditions of overexpression, in which it has been shown to aggregate in discrete nuclear foci. However, these GFP-tagged Rad51 proteins are not fully functional, and thus are not suitable for studies aimed at probing the mechanisms and protein partners of Rad51 in DNA repair in HR and ALT.

Hence, there is a need for identifying new tools for studying DNA-repair mechanisms, notably their *in vivo* dynamics, and developing new treatments of cancer.

Inventors have been able to set up a fully functional tagged Rad51 construct which provides a valuable tool for use in these types of studies, notably to study *in vivo* dynamics of DNA repair, and screening drug candidates which target these pathways and constitute valuable candidates for the treatment of cancer and notably cancer resistant to chemotherapeutic treatments and/or radiotherapy.

### SUMMARY

Accordingly, a first object of the invention is a fully functional tagged Rad51 protein characterized in that it comprises a tag between residues 54 and 55 of Rad 51 protein of SEQ ID NO:1 or between corresponding residues of any budding yeast ortholog thereof.

As shown in the experimental section, said tagged Rad51 is found to be fully functional in regard to the DNA repair mechanisms implying Rad51 protein, and therefore constitutes a valuable tool for studying said mechanisms.

According to other optional features of the tagged Rad51 according to the invention, it can optionally include one or more of the following characteristics alone or in combination:
- the tag is a protein tag, indeed such tags are particularly convenient to be inserted within proteins sequences;
- the tag is a fluorescent protein tag selected from mTagBFP2, TagBFP2, mTurquoise2, mCerulean3, EGFP, mEOS3.2, mWasabi, Superfolder GFP (sfGFP), mNeonGreen, mClover3, Venus, Citrine, mKOκ, tdTomato, TagRFP-T, mRuby3, mScarlet, FusionRed, mStable, mKate2, mMaroon1, mCardinal, T-Sapphire, mCyRFP1, LSSmOrange, mBeRFP, CreiLOV, UnaG, miRFP670, TDsmURFP, iRFP670, mIFP, iFP2.0, iRFP720 EBFP, ECFP, yEGFP, YFP, mHoneydew, mOrange, mTangenine, mStrawberry, mCherry, MGrape1, mRaspberry, mGrape2, mPlum or Halo, such tag allows to follow in real time the structure implying Rad51 protein in yeast and their dynamics of formation;

- the protein tag is flanked by peptide linkers, each of at least 11 amino acids long, indeed this allows the tag to be properly exposed outside the three-dimensional folded Rad51 protein, and out of the structures formed by Rad51, therefore resulting in an improved functionality of the Rad51 ;
- said peptide linkers comprise at least 1, preferably at least 2, even more preferably at least 3 positively charged amino acid residues at 2 to 4 positions from anchoring residues of the protein tag ; linkers with said features allow a better presentation of the tag;
- the linker in Nter of the protein tag is of sequence SEQ ID 9 and the linker in Cter of the protein tag is of sequence SEQ ID 10 or wherein the linker in Nter of the protein tag is of sequence SEQ ID 11 and the linker in Cter of the protein tag is of sequence SEQ ID 12; said peptide linkers allow a better Rad51 activity;

A second object of the invention is a cell comprising a nucleic acid encoding for a fully functional tagged Rad51 protein of a budding yeast characterized in that it comprises a tag between residue 54 and 55 of Rad51 protein of SEQ ID 1 or between corresponding residues of any budding yeast ortholog thereof. Such a cell can be used as a tool for, e.g., screening methods a compound of interfering with homologous recombination of DNA, or for example to produce fully functional tagged Rad51 protein according to the first object of the invention, for its use in *in vitro* assays.

A third object of the invention is a method for screening for a candidate compound capable of interfering with homologous recombination of DNA upon double strand break repair and/or in the alternative lengthening of telomere recombination pathway involving Rad51, said method comprising:
- contacting with said candidate compound a cell comprising a nucleic acid encoding for a eukaryotic fully functional tagged Rad51 protein characterized in that it comprises a protein fluorescent tag between residues 54 and 55 of Rad51 protein of SEQ ID NO 1 or between corresponding residues of any budding yeast ortholog thereof,
- analysing in said cell at least one type of structure comprising the tagged Rad51 without and upon double strand break induction,
- selecting a candidate compound which induces, when compared to at least one control condition, an alteration in said at least one type of structure comprising the tagged Rad51 protein, as a candidate compound capable of interfering with homologous recombination of DNA upon double strand break repair and/or in the alternative lengthening of telomere recombination pathway involving Rad51.

Indeed, the commonly used N terminally tagged Rad51 proteins of the art is poorly functional, as shown e.g. by its sensitivity to methyl methanesulfonate genotoxic stress. Also, the method according to the invention, is likely to allow identifying compounds which will more likely and effectively interfere with the actual mechanisms of homologous recombination of DNA upon double strand break repair and/or in the alternative lengthening of telomere recombination pathway involving Rad51. Such compounds themselves provide valuable tools for studying homologous recombination of DNA upon double strand break repair and/or in the alternative lengthening of telomere recombination pathway and also drug candidates for treating diseases wherein it is desirable to improve or, conversely impede, said mechanisms.

According to other optional features said method according to the third object of the invention can optionally include one or more of the following characteristics alone or in combination:
- in said method, the at least one type of structure comprises a filament, foci, globular structures comprising said tagged Rad51 protein. These features, related to the functional tagged Rad 51 protein of the invention were not observed using N terminally tagged protein, or with other tools, because of the poor functionality of the N terminally tagged protein, and/or the lability of said structures;
- the alteration comprises a deviation, when compared to the at least one control condition, in the number, the shape, the brightness and/or the dynamics of said at least one type of structure;
- the alteration comprises an increase of the rate of the compaction/extension cycles of Rad51 filaments, with an average contraction event occurring in a time laps of more than 20 min. This time laps results in a less effective homologous recombination in DNA repair mechanism;
- the alteration comprises a decrease of the length of the filament that is formed upon double strand break induction. This results in a less effective homologous recombination of DNA repair mechanism;

A fourth object of the invention is a computer implemented method of analysing homologous recombination of DNA upon double strand break repair mechanism and/or the alternative lengthening of telomere recombination pathway involving Rad51, comprising:
- detecting, in at least one image of at least one cell comprising a nucleic acid encoding for a fully functional tagged Rad51 protein of a budding yeast characterized in that it comprises a fluorescent protein tag between the residues 54 and 55 of Rad51 protein of SEQ ID 1 or between corresponding residues of any budding yeast ortholog, at least one type of structure comprising said Rad51 protein tagged with a fluorescent protein between residues 54 and 55, and/or with or without at least one short telomere,
- determining the number, the shape, the brightness and/or the dynamics of said at least one type of structure,
wherein said number, shape, brightness and/or the dynamics of said at least one type of structure characterizes, in said at least one cell, homologous recombination of DNA upon double strand break repair mechanism and/or the alternative lengthening of telomere recombination pathway involving Rad51.

Such a method if particularly suitable for establishing a statistical analysis and monitoring cellular processes implying Rad51 protein which are required to perform analysis on a representative number of cells and on a significant experiment duration.

A fifth object of the invention is a method for screening for a candidate compound capable of sensitizing a cancer cell to a chemotherapeutic agent and/or exposure to radiations comprising:
- contacting with said candidate compound a cell comprising a nucleic acid encoding for a fully functional tagged Rad51 protein characterized in that it comprises a protein fluorescent tag between s 54 and 55 of Rad51 protein of SEQ ID NO: 1 or between corresponding residues of any budding yeast ortholog thereof,
- analysing in said cell at least one type of Rad51 structure without and upon double strand break induction, and/or with or without at least one short telomere,
- selecting a candidate compound which induces an alteration in said at least one type of structure comprising the tagged Rad51 protein as a candidate compound capable of interfering with homologous recombination of DNA upon double strand break repair and/or in the alternative lengthening of telomere recombination pathway involving Rad51,
- contacting at least one cancer cell with the compound selected in preceding step before or concomitantly to a treatment with a chemotherapeutic agent and/or ionising radiation,
if an increase in the death rate of cancer cells is reported in regard to control cancer cells not contacted with the selected compound and submitted to said treatment with a chemotherapeutic agent or ionising radiation, then selecting said compound as a compound capable of sensitizing a cancer cell to chemotherapeutic agent and/or radiotherapy.

Resistance of cancer cells to DNA-damaging radiation and chemotherapy is linked to a deregulation in these cells of the homologous recombination DNA repair mechanism and/or of the alternative lengthening of telomere recombination pathway. Also, because the tagged Rad51 protein of the invention is fully functional it allows, when used in the above method, the identification of compounds more likely to be effective in cancer treatment and or in sensitizing a cancer cell to ionising radiation and/or chemotherapy.

A sixth object of the invention is the *in vitro* or *in cellulo* use of a fully functional tagged Rad51 protein characterized in that it comprises a tag between residues 54 and 55 of Rad51 protein of SEQ ID 1 or between corresponding residues of any budding yeast ortholog thereof, as a reporter of DNA homologous recombination upon double strand break repair mechanism and/or of the alternative lengthening of telomere recombination mechanism involving Rad51. As shown in the experimental section, being fully functional, the tagged Rad51 according to the invention allows, for example, to more accurately monitor said mechanisms.

### FIGURE LEGENDS

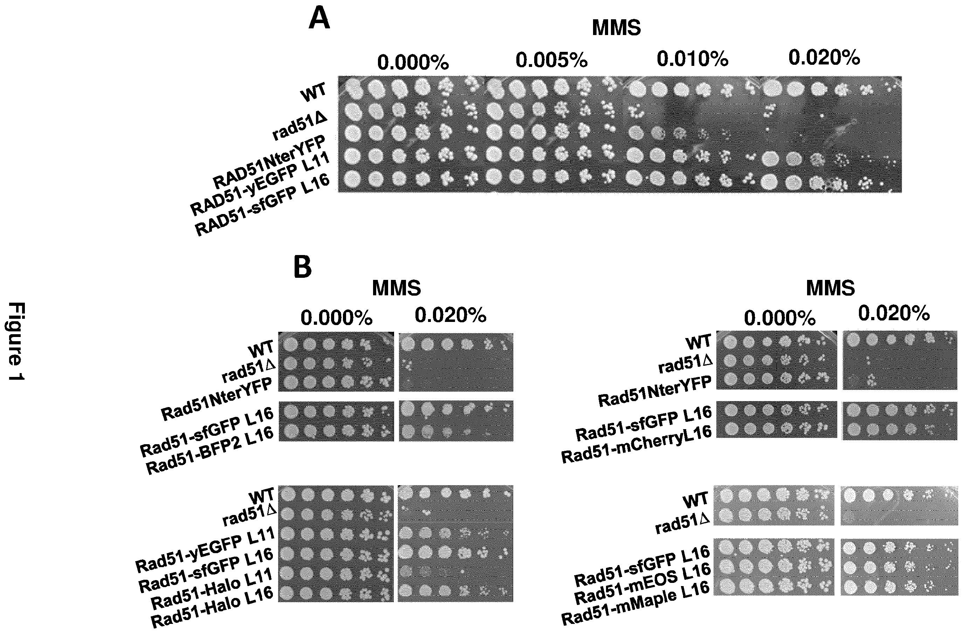

Figure 1: Fully functional Tagged Rad51 **A.** Sensitivity of WT, rad51Δ, N-terminally tagged YFP-Rad51, and internally tagged Rad51 -sfGFP or -yEGFP according to the invention to the genotoxic agent Methyl Methane Sulfonate (MMS), with 11 aa or 16 aa long linkers (respectively L11 and L16). **B.** MMS sensitivity assay. of internally tagged Rad51-Halo, -TagBFP2 (BFP2), -mCherry, - mEOS 3.2 (mEOS) or -mMaple with 11 aa or 16 aa long linkers (respectively L11 and L16).

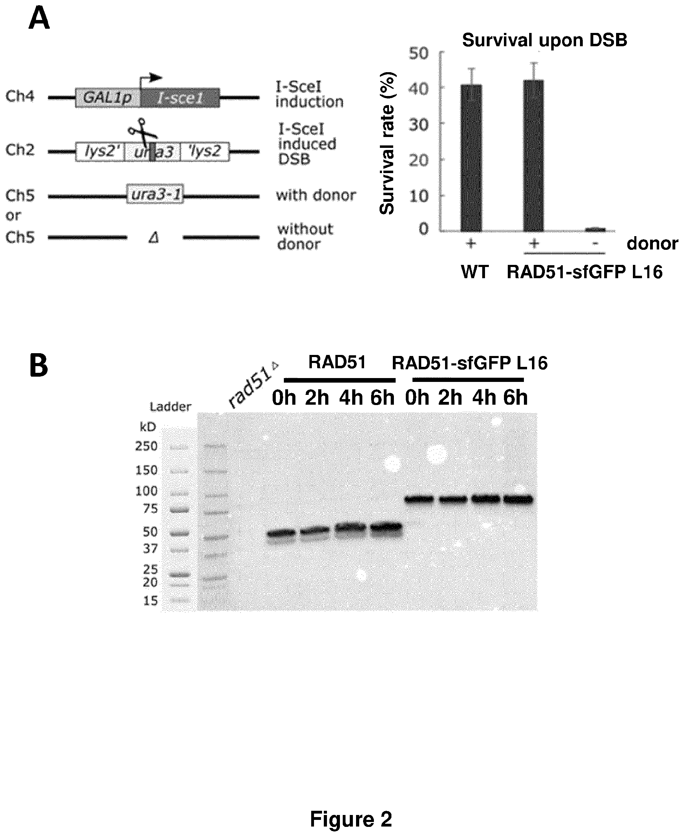

Figure 2: Fully functional Tagged Rad51 **A.** Gene conversion assay. Left panel: Schematic description of the galactose induced DSB by I-Scel ; right panel: survival rate of the untagged or Rad51-sfGFP L16 strain upon DSB induction (galactose plate) in the presence or absence of a donor sequence. **B.** Western blot quantification of RAD51 and of an internally tagged Rad51-sfGFP L16 according to the invention expressed in W303 genetic background.

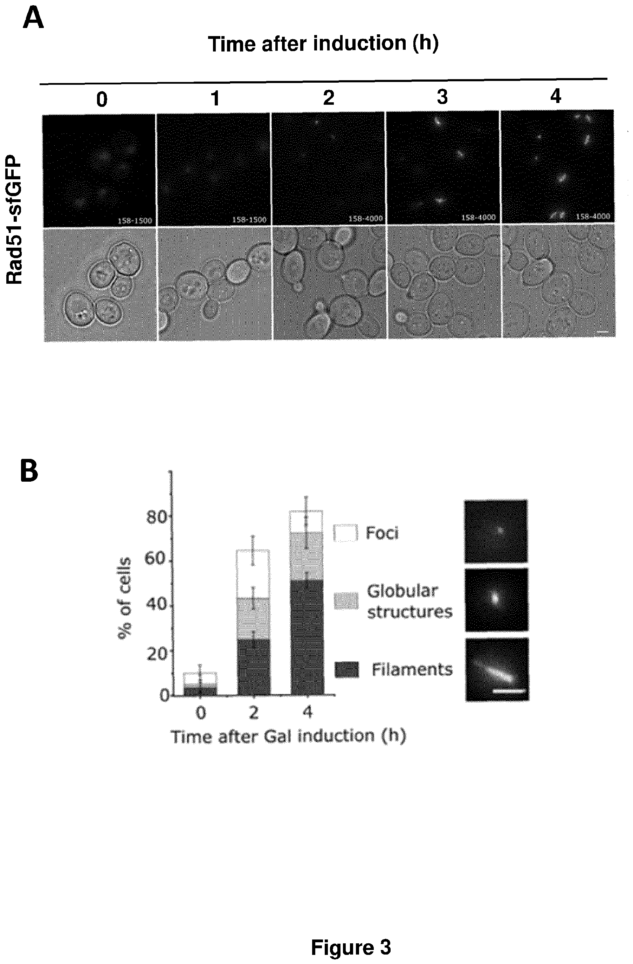

Figure 3: Rad51 forms filaments upon DSB to perform homology search in living cells **A.** Representative images (Transmitted-light image and the GFP channel fluorescent image) of a Rad51-sfGFP L16 expressing strain according to the invention at different times after DSB induction. Z-projection is applied on fluorescent images. **B.** Percentages of internally tagged Rad51 foci, globular structures, and filaments at different times after DSB induction.

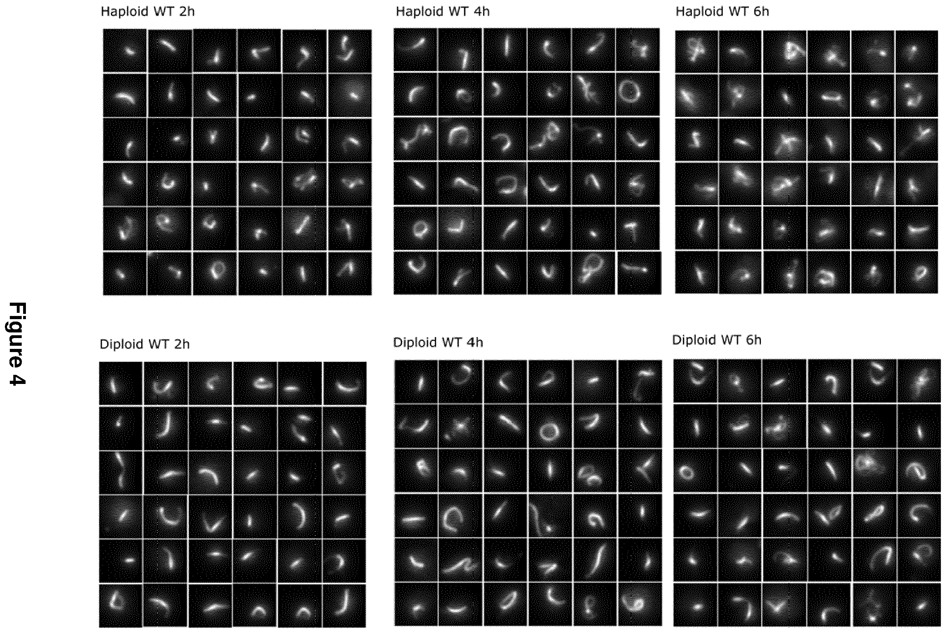

Figure 4: Rad51 filaments form different shapes. Representative filaments of WT haploid and diploid strains at a different time after DSB induction, in the Gene conversion experiments.

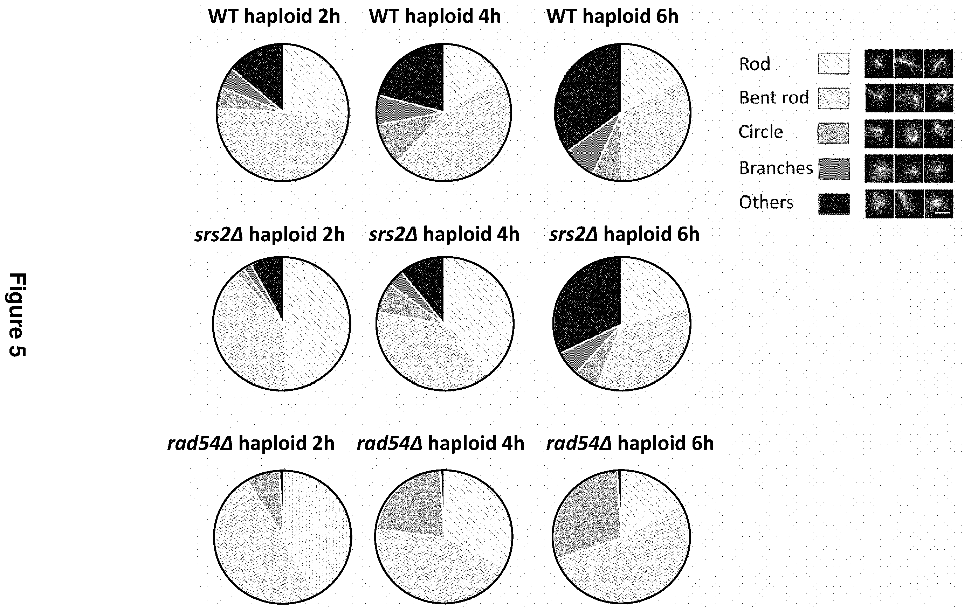

Figure 5: Distributions of Rad51 substructure changes upon interference with Homologous Recombination (HR) repair mechanism.

### DETAILED DESCRIPTION OF THE INVENTION

### Definitions

As exposed above, "Rad51" is a protein which is highly conserved in most eukaryotes and implied in repair of DNA double strand breaks by homologous recombination and in Alternative Lengthening of Telomeres (ALT). Human RAD51 ortholog is also found to interact with BRCA1 and BRCA2 which may be important for the cellular response to DNA damage. Rad51 is known as the homologous of bacterial RecA protein. In budding yeast, Rad51 is encoded by the *RAD51* gene (also known as *YER095W* or *MUT5*). Amino acid sequence of S288C strain Rad51 (SEQ ID NO:1) is:

“MSQVQEQHISESQLQYGNGSLMSTVPADLSQSVVDGNGNGSSEDIEATNGSGDGGGLQEQA
EAQGEMEDEAYDEAALGSFVPIEKLQVNGITMADVKKLRESGLHTAEAVAYAPRKDLLEIKGISE
AKADKLLNEAARLVPMGFVTAADFHMRRSELICLTTGSKNLDTLLGGGVETGSITELFGEFRTG
KSQLCHTLAVTCQIPLDIGGGEGKCLYIDTEGTFRPVRLVSIAQRFGLDPDDALNNVAYARAYN
ADHQLRLLDAAAQMMSESRFSLIVVDSVMALYRTDFSGRGELSARQMHLAKFMRALQRLADQ
FGVAVVVTNQVVAQVDGGMAFNPDPKKPIGGNIMAHSSTTRLGFKKGKGCQRLCKVVDSPCL
PEAECVFAIYEDGVGDPREEDE”.

Except otherwise stated, all peptide sequences are displayed following the writing convention for peptide sequences, from the N-terminal (Nt) end to the C-terminal (Ct) end.

A "budding yeast ortholog" of Rad51 is a budding yeast protein which displays the Rad51 functions. More particularly, it's a budding yeast protein whose sequence shares at least 80% homology with the Rad51 protein of SEQ ID NO:1, while retaining the Rad51 functions, e.g. whose null mutants are sensitive to DNA damaging agents, DNA synthesis and topoisomerase inhibitors to X-rays, gamma rays and/or UV radiation, and/or show increased chromosome instability, and/or show increased loss-of-heterozygosity in diploids. Preferably, the amino acid sequences of the orthologs of the Rad51 protein are identical at more than 80%, preferably more than 81%, more preferably more than 82%, more preferably more than 83%, more preferably more than 84%, more preferably more than 85%, preferably more than 86%, more preferably more than 87%, more preferably more than 88%, more preferably more than 89%, more preferably more than 90%, more preferably more than 91%, more preferably more than 92%, more preferably more than 93%, more preferably more than 94%, more preferably more than 95%, more preferably more than 96% more preferably more than 97% more preferably more than 98% and more preferably more than 99% to SEQ ID NO:1. Preferably, amino acid sequence identity is measured by using the global alignment algorithm of Needleman and Wunsch (1970).

Also, a fully functional Rad51 protein is deemed to have an, at least in part, active DNA double strand breaks repair system. In other words, a fully functional Rad51 protein is functional for homologous recombination. For example, said fully active Rad51 protein:
- allows wild type resistance to a DNA double-strand break (DSB) inducing agents when expressed as the only copy of RAD51 in yeast (testing resistance to DNA double-strand break (DSB) agent is well known in the field, an exemplary protocol for methyl methanesulfonate (MMS) is provided in the experimental section below) and/or,
- is as competent as the wild-type version to perform gene conversion following the induction of unique DSB in the presence of a homologous donor sequence located on a different chromosome (such a test is well known in the field, an exemplary protocol for such a test is provided in the experimental section below) and/or,
- is not affected in its expression level upon the induction of unique DSB in the presence of homologous donor sequence located on a different chromosome (such a test is well known in the field, an exemplary protocol for such a test is provided in the experimental section below).

Except otherwise stated, "Rad51" or "RAD51" or "Rad51 protein" as used herein aims at designating any budding yeast Rad51 protein as defined above.

As used herein budding yeast aims at defining a yeast in opposition to fission yeasts, that divides through a process in which smaller daughter cells pinch, or bud, off the mother cell. In particular, budding yeasts comprise yeasts of *Saccharomyces cerevisiae* species, or of genera *Candida* or *Cryptococcus.* More preferably, "budding yeast" designates yeast of *Saccharomyces cerevisiae* species.

A tag is defined as an element that is comprised in a protein and allows said protein being isolated and/or detected. In particular, said tag is not a part of the protein as naturally expressed by the organism it comes from. A tag can be of different nature, for example either a saccharide, a protein, an organic molecule etc... Affinity tags help in protein purification and identification and allow to separate a protein or looking for partners and/or complexes incorporating the protein which is studied. Examples of affinity tags include chitin binding protein (CBP), Maltose Binding Protein (MBP), Strep-tag, glutathione-S-transferase (GST) or poly (HIS) tag ; these tags can be used in chromatography techniques. Epitope tags are usually short peptides recognized by high affinity antibodies. Fusion tags refer to peptide tags that are fused to the peptide sequence of the protein. Fusion tags can be polypeptides of various size and even small proteins or enzymes. Tagging of an endogenous protein in an organism can be done *via* cloning into vectors and using molecular tools well known from the skilled in the art or added in the genome using CRISPR-Cas9 gene editing system.

Up to now, no fully functional tagged Rad51 protein exists. Also, the use and results obtained from the tagged Rad51 of the art (most with a tag at their N-terminal or C-terminal parts) are tainted by doubts about their actual significance about *in vivo* mechanisms implying Rad51 in *e.g.* homologous recombination during double strand break repair. Also, because its implication in different cancers (Rad51 being found over expressed or under expressed in different cancers, and identified, for example, as a partner of BRCA2), a fully functional tagged Rad51 protein is useful at deciphering the homologous DNA repair and/or telomere maintenance mechanisms in eukaryotic cells, as well as mechanisms underlying the resistance of cancer cells to DNA damages, *i.e.* resistance to radiotherapy and/or to chemotherapeutic agents. Such a protein is useful also as a tool for screening drug candidates for treating cancer, and/or sensitizing cancer cells to genotoxic agents responsible for double strand breaks.

### Fully functional tagged Rad51 protein

Accordingly, in a first aspect, the invention relates to a fully functional tagged Rad51 protein. Through an extensive study of the structure of several Rad51 protein, and several attempts, inventors have identified a location within the Rad51 protein sequence, wherein the introduction of a tag would be likely of a less detrimental on the functions of Rad51 in cells. This location lies between residues 44 and 65 of SEQ ID NO:1. More particularly, as it will be further detailed below, the site between Gly 54 and Gly 55 is particularly suitable and allows he introduction of various tags with no effects on the functions of Rad51 in cells. This is particularly surprising because up to now, N terminally- or C terminally-tagged (with so-called external tag) version of Rad51 were found poorly functional, and it could therefore be inferred that any modification of protein, even outside the native sequence, is sufficient to disturb or disrupt the complexes implied in rad51 dependent DNA repair mechanisms.

In a preferred embodiment, said fully active Rad51 protein allows wild type resistance to a DNA double-strand break (DSB) inducing agents when expressed as the only copy of *RAD51* in yeast (as determined, e.g. by an MMS viability assay).

In another preferred embodiment said fully active tagged Rad51 protein is as competent as the wild-type version to perform gene conversion following the induction of unique DSB in the presence of a homologous donor sequence located on a different chromosome (such a test is well known in the field, an exemplary protocol for such a test is provided in the experimental section below).

In another preferred embodiment said fully active tagged Rad51 protein is not affected in its expression level upon the induction of unique DSB in the presence of homologous donor sequence located on a different chromosome (such a test is well known in the field, an exemplary protocol for such a test is provided in the experimental section below).

In a particularly preferred embodiment, said fully active tagged Rad51 protein:
- allows wild type resistance to a DNA double-strand break (DSB) inducing agents when expressed as the only copy of RAD51 in yeast, and
- is as competent as the wild-type version to perform gene conversion following the induction of unique DSB in the presence of a homologous donor sequence located on a different chromosome, and
- is not affected in its expression level upon the induction of unique DSB in the presence of homologous donor sequence located on a different chromosome.

In another preferred embodiment, said fully active tagged Rad51 protein is as competent as the wild-type version to perform ectopic gene conversion using a homologous donor sequence located on a different chromosome (in donor tests as exposed in the experimental section).

More particularly, inventors have identified that the site between Gly 54 and Gly 55 of the Rad51 of SEQ ID NO:1 is the more favorable insertion site for a tag and contributes to obtain a fully functional tagged Rad51 protein, with a so-called internal tag.

Tag of any type known in the art, e.g., as defined above, can be inserted at this position, depending of the assay that is contemplated, provided that the tagged Rad51 protein is functional for homologous recombination, as determined, for example, using at least one of the tests as mentioned above. More preferably, said tagged Rad51 protein fulfills the three tests as disclosed above. The internal tag can be a saccharide, a protein, an organic molecule etc.... More preferably, said tag is a protein tag comprising a peptide sequence. As shown in the experimental section, the site between Gly 54 and Gly 55 of the Rad51 of SEQ ID NO:1 is a particular place where it is possible to use many different protein tags in length and/or types with no or few detrimental effects to Rad51 functionality.

In a particular embodiment said protein tag is an affinity tag which are well known in the art, for example selected from chitin binding protein (CBP), maltose binding protein (MBP), Strep-tag, glutathione-S-transferase (GST) or poly (HIS) tag and Myc tag.

In another particular embodiment, said protein tag is an epitope tag, useful for example in western blotting, immunofluorescence or immunoprecipitation experiments.

In another embodiment, said protein tag is an auxin inducible degron tag (AID tag), which allows controlling protein level *in vivo* through the inducible rapid depletion of the tagged protein upon exposure to auxin. Such tags can be expanded by any epitope tags or other tags such as those, but not only, described herein.

In another particular embodiment, said protein tag is a fluorescent tag as listed for example in Thorn (2017). Fluorescent tags are useful, for example, for obtaining a readout of the protein in living cells. Fluorescent protein tag can be an extrinsically fluorescent proteins that bind either an endogenous or an exogenous biomolecule as a fluorophore, such as for example, Halo, FlAsH or ReAsH tags. Particularly preferred fluorescent protein tag are intrinsically fluorescent tags, which become fluorescent upon folding without addition of a fluorophore.

More particularly said fluorescent tag can be selected from mTagBFP2, TagBFP2, mTurquoise2, mCerulean3, EGFP, mEOS3.2, mWasabi, Superfolder GFP (sfGFP), mNeonGreen, mClover3, Venus, Citrine, mKOκ, tdTomato, TagRFP-T, mRuby3, mScarlet, FusionRed, mStable, mKate2, mMaroon1, mCardinal, T-Sapphire, mCyRFP1, LSSmOrange, mBeRFP, CreiLOV, UnaG, miRFP670, TDsmURFP, iRFP670, mIFP, iFP2.0, iRFP720 EBFP, ECFP, yEGFP, YFP, , mHoneydew, mOrange, mTangenine, mStrawberry, mCherry, MGrape1, mRaspberry, mGrape2, mPlum or Halo. Preferably said fluorescent tag is selected from GFP, BFP, EBFP, ECFP, EGFP, yEGFP, sfGFP, YFP, mHoneydew, mOrange, tdTomato, mTangenine, mStrawberry, mCherry, MGrape1, mRaspberry, mGrape2, mMaple, mEOS3.2, mTagBFP2, TagBFP2, Halo or mPlum.

Green fluorescent protein (GFP) and its derivatives are particularly preferred. In another very particular embodiment said fluorescent tag is selected from GFP, EGFP, sfGFP, yEGFP, mCherry, mMaple, mEOS3.2, mTagBFP2, TagBFP2, Halo protein tags.

Amino acid sequence of a particularly preferred mMaple used as a protein tag as exposed above is SEQ ID NO:2 :

“VSKGEETIMSVIKPDMKIKLRMEGNVNGHAFVIEGEGSGKPFEGIQTIDLEVKEGAPLPFAYDIL
TTAFHYGNRVFTKYPEDIPDYFKQSFPEGYSWERSMTYEDGGICIATNDITMEEDSFINKIHFKG
TNFPPNGPVMQKRTVGWEVSTEKMYVRDGVLKGDVKMKLLLKGGSHYRCDFRTTYKVKQKA
VKLPDYHFVDHRIEILSHDKDYNKVKLYEHAVARNSTDSMDELYK”.

Amino acid sequence of a particularly preferred mEOS3.2 used as a protein tag as exposed above is SEQ ID NO:3 :

“MSAIKPDMKIKLRMEGNVNGHHFVIDGDGTGKPFEGKQSMDLEVKEGGPLPFAFDILTTAFHY
GNRVFAKYPDNIQDYFKQSFPKGYSWERSLTFEDGGICNARNDITMEGDTFYNKVRFYGTNFP
ANGPVMQKKTLKWEPSTEKMYVRDGVLTGDIEMALLLEGNAHYRCDFRTTYKAKEKGVKLPG
AHFVDHCIEILSHDKDYNKVKLYEHAVAHSGLPDNARR”.

Amino acid sequence of a particularly preferred superfolder GFP (sfGFP) used as a protein tag as exposed above is SEQ ID NO:4 :

“MSKGEELFTGVVPILVELDGDVNGHKFSVRGEGEGDATNGKLTLKFICTTGKLPVPWPTLVTT
LTYGVQCFSRYPDHMKRHDFFKSAMPEGYVQERTISFKDDGTYKTRAEVKFEGDTLVNRIELK
GIDFKEDGNILGHKLEYNFNSHNVYITADKQKNGIKANFKIRHNVEDGSVQLADHYQQNTPIGD
GPVLLPDNHYLSTQSVLSKDPNEKRDHMVLLEFVTAAGITHGMDELYK”.

Amino acid sequence of a particularly preferred yEGFP used as a protein tag as exposed above is SEQ ID NO:5 :

“SKGEELFTGVVPILVELDGDVNGHKFSVSGEGEGDATYGKLTLKFICTTGKLPVPWPTLVTTFG
YGVQCFARYPDHMKQHDFFKSAMPEGYVQERTIFFKDDGNYKTRAEVKFEGDTLVNRIELKGI
DFKEDGNILGHKLEYNYNSHNVYIMADKQKNGIKVNFKIRHNIEDGSVQLADHYQQNTPIGDGP
VLLPDNHYLSTQSALSKDPNEKTDHMVLLEFVTAAGITHGMDELYK”.

Amino acid sequence of a particularly preferred mCherry used as a protein tag is SEQ ID NO:6 :

“VSKGEEDNMAIIKEFMRFKVHMEGSVNGHEFEIEGEGEGRPYEGTQTAKLKVTKGGPLPFAW
DILSPQFMYGSKAYVKHPADIPDYLKLSFPEGFKWERVMNFEDGGVVTVTQDSSLQDGEFIYK
VKLRGTNFPSDGPVMQKKTMGWEASSERMYPEDGALKGEIKQRLKLKDGGHYDAEVKTTYKA
KKPVQLPGAYNVNIKLDITSHNEDYTIVEQYERAEGRHSTGGMDELYK”.

Amino acid sequence of a particularly preferred TagBFP2 used as a protein tag as exposed above is SEQ ID NO:7 :

“MSELIKENMHMKLYMEGTVDNHHFKCTSEGEGKPYEGTQTMRIKVVEGGPLPFAFDILATSFL
YGSKTFINHTQGIPDFFKQSFPEGFTWERVTTYEDGGVLTATQDTSLQDGCLIYNVKIRGVNFT
SNGPVMQKKTLGWEAFTETLYPADGGLEGRNDMALKLVGGSHLIANAKTTYRSKKPAKNLKM
PVYYVDYRLERKEANNETYVEQHEVAVARYCDLPSKLGHKLN”.

In another particular embodiment said tag is an affinity tag. For example, such affinity tag can be used to capture and purify protein complexes comprising Rad51 and Rad51 partners. In a particular embodiment, a Halo tag is used in the tagged Rad51 protein.

Amino acid sequence of a particularly preferred Halo used as a protein tag as exposed above is SEQ ID NO:8:

“MAEIGTGFPFDPHYVEVLGERMHYVDVGPRDGTPVLFLHGNPTSSYVWRNIIPHVAPTHRCIA
PDLIGMGKSDKPDLGYFFDDHVRFMDAFIEALGLEEVVLVIHDWGSALGFHWAKRNPERVKGI
AFMEFIRPIPTWDEWPEFARETFQAFRTTDVGRKLIIDQNVFIEGTLPMGVVRPLTEVEMDHYR
EPFLNPVDREPLWRFPNELPIAGEPANIVALVEEYMDWLHQSPVPKLLFWGTPGVLIPPAEAAR
LAKSLPNCKAVDIGPGLNLLQEDNPDLIGSEIARWLSTLEISG”.

As stated above Halo tag can be used as a fluorescent tag or as an affinity tag. It has been also discovered that obtaining a fully active Rad51 protein as defined above is favored by using linker peptides upstream and downstream the internal protein tag. Preferably said upstream and downstream linkers are of at least 11 amino acids long. In a particular embodiment, said downstream and upstream linkers are, independently of each other, of between 11 to 50, 11 to 40, 11 to 30, 11 to 20, or more preferably of between 11 to 16 amino acids long.

Said linker should be such as favoring the exposure of the protein tag outside of the structure Rad51 protein without interfering with Rad51 capacity to cover ssDNA.

In a particular embodiment, said upstream and downstream linkers count the same number of amino acid residues.

In a more particular embodiment, amino acid sequences of downstream and upstream linker are identical. In a particular embodiment, said upstream and downstream linker are 11 amino acid long and of sequence: SEQ ID NO: 9 "GGAGSAGGAGG".

In a yet preferred embodiment, linkers are of sequences and count the same number of amino acid residues and display opposite Nt to Ct sequences. Accordingly, in a particular embodiment an upstream linker is of sequence SEQ ID NO: 9 "GGAGSAGGAGG" and downstream linker is of sequence SEQ ID NO: 10 "GGAGGASGAGG".

Advantageously, without wishing to be bound by any theory, the number of charges in the linkers should balance the net difference between negatively and positively charged residues present at the surface of the inserted tag in a distance of 15 Å from the Cα of the anchoring residue (the linker residues at the junction with residues of Rad51 protein). Indeed, this is thought to favor an optimal exposure of the tag outside of the folded Rad51 protein or of cellular structures comprising tagged Rad51 protein.

In an embodiment, in case where the tag is positively charged, the linker comprises negatively charged amino acid residues. More advantageously, the number of positive charges in the linkers balance the net difference between negatively and positively charged residues present at the surface of the inserted tag in a distance of 15 Å from the Cα of the anchoring residues. Also, in a particular embodiment, linkers comprise at least 1, preferably at least 2, even more preferably at least 3 negatively charged amino acid residues at 2 to 4 positions from anchoring residues.

In another embodiment, in case where the tag is negatively charged, the linker comprises positively charged amino acid residues. More advantageously, the number of positive charges in the linkers should balance the net difference between negatively and positively charged residues present at the surface of the inserted tag in a distance of 15 Å from the Cα of the anchoring residues. Also, in a particular embodiment, linkers comprise at least 1, preferably at least 2, even more preferably at least 3 positively charged amino acid residues at 2 to 4 positions from anchoring residues.

In a preferred embodiment said upstream linker is of sequence [GAS]{12,47}-[RK]{1 ,3}-[GAS] and downstream linker is of sequence [GAS]-[RK]{1,3}-[GAS]{12,47}. This means that, in this embodiment, a 16-50 amino acid long upstream linker comprises, from Nt to Ct, from 12 to 47 amino acids chosen between G, A, S, from 1 to 3 amino acids chosen between R and K and one amino acid residue chosen between G, A and S. Accordingly, a 16-50 amino acid long downstream linker comprises, from Nt to Ct, one amino acid residue chosen between G, A and S, followed by from 1 to 3 amino acids chosen between R and K and from 12 to 47 amino acids chosen between G, A, S.

In a yet preferred embodiment said upstream ([GAS]{12,47}-[RK]{1,3}-[GAS]) and downstream ([GAS]-[RK]{1 ,3}-[GAS]{12,47})linkers count the same number of amino acid residues and display opposite Nt to Ct sequences. More particularly the upstream linker is of sequence SEQ ID NO: 11 "GGAGSAGGAGSSRKRS" and downstream linker is of sequence SEQ ID NO: 12 "SRKRSSGAGSAGGAGG".

In a particularly preferred embodiment of the fully functional tagged Rad51 protein according to the invention, upstream linker [GAS]-{12,46}-[RK]-{1,3}-[GAS] and downstream linker [GAS]-{1,3}-[RK]-{12,46}-[GAS] present the same length in amino acid residues. This participates to the convenient presentation of the tag in Rad51 protein structures. More particularly, in this embodiment, upstream and downstream linkers are of sequences SEQ ID NO: 11 and SEQ ID NO: 12 respectively. In an even more particular embodiment, upstream and downstream linkers are of sequences SEQ ID NO: 11 and SEQ ID NO: 12 and the protein tag is a sfGFP. In another particular embodiment, upstream and downstream linkers are of sequences SEQ ID NO: 11 and SEQ ID NO: 12 and the protein tag is a mMaple. In another particular embodiment, upstream and downstream linkers are of sequences SEQ ID NO: 11 and SEQ ID NO: 12 and the protein tag is a mEOS3.2. In another particular embodiment, upstream and downstream linkers are of sequences SEQ ID NO: 11 and SEQ ID NO: 12 and the protein tag is a yEGFP. In another particular embodiment, upstream and downstream linkers are of sequences SEQ ID NO: 11 and SEQ ID NO: 12 and the protein tag is a mCherry. In another particular embodiment, upstream and downstream linkers are of sequences SEQ ID NO: 11 and SEQ ID NO: 12 and the protein tag is TagBFP2. In another particular embodiment, upstream and downstream linkers are of sequences SEQ ID NO: 11 and SEQ ID NO: 12 and the protein tag is a Halo.

As shown in the experimental section, a fully functional tagged Rad51 protein according to the invention is a valuable and accurate *in vitro* (e.g. not in a living cell) or *in cellulo* reporter of DNA homologous recombination upon double strand break repair mechanism and/or of the alternative lengthening of telomere recombination mechanism involving Rad51. More particularly, said protein, when expressed as the only Rad51 protein, for example in different mutants for e.g. potential interacting partners of Rad51, is a valuable tool for deciphering the actual role of the potential interaction in DNA homologous recombination upon double strand break repair mechanism and/or of the alternative lengthening of telomere recombination mechanism involving Rad51. Also the fully functional tagged Rad51 protein as described above is a valuable tool for *in vitro* or *in cellulo* assays or experiment based on Rad51.

In a particular embodiment, a fully functional tagged Rad51 protein is of the following sequence ID NO: 13 :

“MSQVQEQHISESQLQYGNGSLMSTVPADLSQSVVDGNGNGSSEDIEATNGSGDGGGAGSA
GGAGNRKRNGMSKGEELFTGVVPILVELDGDVNGHKFSVRGEGEGDATNGKLTLKFICTTGKL
PVPWPTLVTTLTYGVQCFSRYPDHMKRHDFFKSAMPEGYVQERTISFKDDGTYKTRAEVKFE
GDTLVNRIELKGIDFKEDGNILGHKLEYNFNSHNVYITADKQKNGIKANFKIRHNVEDGSVQLAD
HYQQNTPIGDGPVLLPDNHYLSTQSVLSKDPNEKRDHMVLLEFVTAAGITHGMDELYKGNRKR
NGAGSAGGAGGGGLQEQAEAQGEMEDEAYDEAALGSFVPIEKLQVNGITMADVKKLRESGLH
TAEAVAYAPRKDLLEIKGISEAKADKLLNEAARLVPMGFVTAADFHMRRSELICLTTGSKNLDTL

LGGGVETGSITELFGEFRTGKSQLCHTLAVTCQIPLDIGGGEGKCLYIDTEGTFRPVRLVSIAQR
FGLDPDDALNNVAYARAYNADHQLRLLDAAAQMMSESRFSLIVVDSVMALYRTDFSGRGELSA
RQMHLAKFMRALQRLADQFGVAVVVTNQVVAQVDGGMAFNPDPKKPIGGNIMAHSSTTRLGF
KKGKGCQRLCKVVDSPCLPEAECVFAIYEDGVGDPREEDE".

In a particular embodiment, a fully functional tagged Rad51 protein is the one expressed by the the clone yAT3879 deposited at Institut Pasteur on 18 November 2022 which is available from the Institute Pasteur (CNCM, 25 rue du Docteur Roux, F-75724 PARIS Cedex 15, France) under deposit No. CNCM I-5918.

### A cell comprising a nucleic acid encoding for a fully functional tagged Rad51 protein according to the invention

In a second aspect the invention relates to a cell which expresses the fully functional tagged Rad51 protein as exposed above. Accordingly said cell comprises a nucleic acid encoding for a fully functional tagged Rad51 protein.

In a particular embodiment, said cell is used to overproduce in a sufficient amount the afore described fully functional tagged Rad51 protein.

In another embodiment, said cell is a eukaryotic cell. In a more particular embodiment, said cells is a budding yeast, preferably a cell of a *Saccharomyces cerevisiae* strain.

In another particular embodiment, said cell is a eukaryotic cell, preferably a budding yeast, more preferably *S. cerevisiae,* and the Rad51 is expressed in said cell as the only copy of Rad51.

In a particular embodiment, said nucleic acid is an expression vector.

Even more preferably, said only copy is localized at the original locus of Rad51 in said eukaryotic cell. More preferably, said eukaryotic cell is a budding yeast, a *Saccharomyces cerevisiae* strain being particularly preferred. Then, nucleic acid encoding said only one copy of Rad51, which codes for the fully functional tagged Rad51 of the invention, is localized at the locus of Rad51 gene in wild type strain of budding yeast and replaces said gene. Said cell can be constructed using biology molecular tools well known from the skilled in the art, for example, the tag can be inserted in Rad51 gene through CRISPR/CAS9 technology, as exemplified below. Such a cell provides a particularly convenient tool to study dynamics and features of Rad51 assemblies in cells exposed to double strand break events.

Clone yAT3879 constitutes a particular embodiment of the cell according to the invention. yAT3879 clone has been deposited at Institut Pasteur on 18 November 2022 and is available from the Institut Pasteur (CNCM, 25 rue du Docteur Roux, F-75724 PARIS Cedex 15, France) under deposit No. CNCM I-5918.

### Methods of screening candidate compounds

As already mentioned, availability of a fully functional tagged Rad51 protein is of the outmost interest as a reporter of the DNA homologous recombination upon double-strand break repair mechanism and/or of the alternative lengthening of telomere recombination mechanism involving Rad51. Compounds capable of interfering with such mechanisms *i.e.* exposure thereto results in either an improvement or worsening of DNA repair and/or lengthening of telomeres mechanisms constitute valuable biotechnical tools to study DNA repair mechanism and/or valuable drug candidates for treating proliferative diseases, and more particularly, cancers.

Indeed, fully functional tagged Rad51 protein according to the invention makes it possible to detect intracellular structures comprising Rad51 as well their dynamics. Also, alteration in said structures and/or their dynamics in cell upon exposure to particular compound or condition is a sign that such a compound or condition is likely interfering with double strand repair and/or telomere lengthening. Accordingly, in a third aspect of the invention relates to a method of screening candidate compounds interfering with double strand repair and/or telomere lengthening, which makes use of a cell which expresses the fully functional tagged Rad51 protein as described above.

Rad51 protein of the invention internally tagged with a fluorescent tag is particularly suitable for such a method, as it allows to follow in real time intracellular structures implying Rad51 as well as their dynamics. A budding yeast cell comprising a nucleic acid comprising a nucleic acid encoding for a fully functional tagged Rad51 protein as defined above is particularly adapted to said method. In a particular embodiment, Clone yAT3879 is used in the method of screening according to the invention.

Said intracellular structures can be selected from the Rad51-ssDNA nucleoprotein filament (known to cover ssDNA), intracellular foci, globular structure, filaments, or specific patterns of Rad51 protein or of their structure within cell. More particularly, as shown in the experimental section, filaments can display several forms (or substructure) that are categorized as: rods, bent filaments, circles, branched structures with a single node and others. Deviations of features of said structures in comparison with control conditions are defined as alterations that can be detected through the method according to the invention. Alterations that can be detected for a structure comprising the tagged Rad51 protein according to the invention range from the presence, the absence, an increase or a decrease in the number of said structures, an increase or a decrease of their size, a change in their shape, their brightness and/or their subcellular localization in comparison with a control condition. An alteration can be also a modification in the distribution (in terms of relative or absolute quantity) of said structures within the nucleus or cell. Also, an alteration can be detected in their dynamics of formation and/or of disappearance within cell, in comparison with a control condition. An alteration can also comprise in lowering or an increase of the proportion, within the whole cell population, of cells displaying a particular structure in comparison with control condition.

A control condition can be a normal condition of culture which allows a normal growth of cells, in absence of any unusual compound or treatment of cells unnecessary to said normal growth. A control condition can be a culture condition comprising the exposure to an inducer of DNA double-strand breaks, such as endonuclease expression, ionizing radiations, radiomimetic chemicals or reactive oxygen species.

Inventors show for the first time the appearance of globular and elongated structures (in the form of filament, different of the Rad 51-ssDNA nucleoprotein filament which covers the ssDNA) upon DSB induction in cell expressing only one copy of Rad51, which codes for the fully functional tagged Rad51 of the invention. As shown in the experimental part such structures are not detected with other tagged Rad51 proteins with a tag graft at their Nt- or Ct- end ;, which are at least in part defective in functional homologous recombination mechanism. Also, in a particular embodiment of the method of screening according the third aspect of the invention, a compound exposure to which results in the absence of said of globular and elongated structure (or filament) despite DSB induction is considered as capable of interfering with DNA homologous recombination upon double-strand break repair mechanism. In another particular embodiment of the method of screening, a compound exposure to which results in a decrease in the number of globular and elongated structure (or filament) in comparison to control condition is considered as capable interfering with DNA homologous recombination upon double-strand break repair mechanism. In another particular embodiment of the method of screening, a compound exposure to which results in a decrease in the number of globular and elongated structure (or filament) in comparison to control condition is considered as capable interfering with DNA homologous recombination upon double-strand break repair mechanism. In another particular embodiment, a compound capable of inducing a deviation in the length of said filaments upon exposure to a compound will be also considered as capable of interfering with DNA homologous recombination upon double-strand break repair mechanism. Further, the Rad51 filaments as identified by the inventors are found to display several patterns such as rods, bent filaments, circles branched structures etc.... Also, a compound exposure to which results in an alteration of said patterns in comparison with a control condition is considered as capable of interfering with DNA homologous recombination upon double-strand break repair mechanism. More particularly, a compound inducing a change in the relative quantity of Rad51 substructures within cells, is considered as capable of interfering with DNA homologous recombination upon double-strand break repair mechanism.

As stated above, the fully functional tagged Rad51 protein according to the invention, more particularly those tagged with a fluorescent tag, are particularly suited to follow the dynamics of the Rad51 structures within cell. Accordingly, in an embodiment of the method of screening, a compound exposure to which results in an alteration of dynamics of said structures is considered as capable of interfering with DNA homologous recombination upon double-strand break repair mechanism, when compared to said dynamics in a control condition. In a particular embodiment, a compaction/extension cycle of a duration of more than 20 minutes, more than 30 minutes, more than 40 minutes, more than 50 minutes, preferably more than 60 minutes is considered as a sign of an alteration of DNA homologous recombination upon double-strand break repair mechanism.

In another particular embodiment, a compaction/extension cycle of a duration of less than 15 minutes, more preferably less than 10 minutes, even less than 5 minutes is considered as a sign of an alteration of DNA homologous recombination upon double-strand break repair mechanism.

Of course, the gene encoding a fully functional tagged Rad51 protein as defined above can be used in a budding yeast of any genetic context or carrying any mutation. For example, in a particular embodiment, the budding yeast cell comprising a nucleic acid encoding for a fully functional tagged Rad51 protein as defined above comprise is a cell with inducible short telomere(s). Yeast strains with inducible short telomeres are well known in the art, as used for example the strain used in Texeira (2013). The skilled in the art will easily know how to implement the systems of inducible short telomere(s) in the methods according to the invention. Such a cell with inducible short telomere(s) and expressing the tagged Rad51 protein of the invention is particularly suitable in the above methods to identify compounds that interfere with the alternative lengthening of telomeres (ALT) recombination pathway. In a particular embodiment, a budding yeast cell of clone yAT3879 is used in the above method of screening for a candidate compound capable of interfering with homologous recombination of DNA upon double strand break repair and/or in the alternative lengthening of telomere recombination pathway involving Rad51.

Compounds identified through the above method as capable of interfering with DNA homologous recombination upon double-strand break repair mechanism and/or of the alternative lengthening of telomere recombination mechanism involving Rad51 are valuable candidates to be used in cancer therapy and more particularly in order to sensitize a cancer cell to a double strand break inducing chemotherapeutic agent and/or exposure to radiations. Indeed, DNA homologous recombination upon double-strand break repair mechanism and the alternative lengthening of telomere recombination mechanism are known to contribute to cancer resistance to treatment. Also, compounds capable to alter such mechanisms provide valuable candidate compounds to be used in treating cancer, more particularly in sensitizing cancer cells to anti-cancer agents inducing DNA double strand break such as chemotherapeutic agent, ionizing radiations or agent inducing Reactive Oxygen Species (ROS) production within cells. Accordingly, the invention relates also to a method of screening for candidate compounds capable of sensitizing a cancer cell to a chemotherapeutic agent and/or exposure to radiations ; said method of screening incorporates the steps of the method as exposed above and further comprises a step of an *in vitro* testing of the compound selected as capable of interfering with homologous recombination of DNA upon double strand break repair and/or in the alternative lengthening of telomere recombination pathway involving Rad51 as on cancer cells. Said *in vitro* testing comprises a step of exposing cancer cells to said selected compound, to determine e.g. their viability, cell cycle duration. In a particular embodiment, said *in vitro* test comprises also exposing cancer cells to (or contacting with) ionizing radiation and/or chemotherapeutic agents. In a particular embodiment step of exposing cancer cell to ionizing radiation and/or therapeutic agent is applied before and/or concomitantly to exposure to the candidate compound. In another particular embodiment, step of exposing cancer cell to ionizing radiation and/or therapeutic agent is applied after and/or concomitantly to exposure to the candidate compound. In another particular embodiment, when the tested compound is found as inducing a loss of viability or a longer cell cycle when compared e.g., to control condition wherein cancer cells are not contacted with said compound, then said compound is selected as capable of sensitizing a cancer cell to a chemotherapeutic agent and/or exposure to radiations. In a particular embodiment, a budding yeast cell of clone yAT3879 is used in the above method of screening of screening for candidate compounds capable of sensitizing a cancer cell to a chemotherapeutic agent and/or exposure to radiations.

### Computer implemented method.

A particular object of the invention is a computer implemented method which comprises the automated analysis of at least one image of a cell expressing a fully functional tagged Rad51 with an internal fluorescent protein tag as described herein. In said method, means are adapted to perform the identification, quantification and/or analysis of the features of the structures comprising the tagged Rad51 protein as listed above. More particularly, said identification, quantification and/or analysis of features can be made through machine learning tools. As shown in the experimental section, said method is particularly adapted to treat a great number of images, to accurately determine the features of Rad51 structures and their dynamics as reporter features of homologous DNA repair mechanism and/or alternative lengthening of telomere efficiency in that cell. More particularly said images can be extracted from a film which is a caption of at least one cell over at least one hour, preferably 2h, 3h, 5h, 6h, 7h, 8h, 9h, 10h, 15h, 20h, 24h or even 48h. in an at least one image of a cell expressing said tagged Rad51 protein.

In a particular embodiment, a budding yeast cell of clone yAT3879 is used in the above method of screening for candidate compounds capable of sensitizing a cancer cell to a chemotherapeutic agent and/or exposure to radiations.

### EXPERIMENTAL DATA

### MATERIAL AND METHODS

### Construction of strain Expressing Rad51 with an internal tag

Strains are derivatives of W303. The internal tagging of Rad51 between the 54th and 55th amino acid was obtained using the CRISPR-Cas9 technique. Strain expressing the Rad51 with internal tagging was obtained by cotransformation of the strain with a plasmid encoding a guide rDNA with a donor sequence that contained *RAD51* homologous sequences on either side of a fluorescent tag flanked by identical linkers (SEQ ID NO: 5, 8 or 9). The donors were generated by PCR on the corresponding protein-tag nucleotide sequence.

### MMS viability assay

Strains were grown overnight in YPD and plated in fivefold serial dilutions starting at OD₆₀₀ₙₘ=1 on YPD plates containing 0%, 0.005%, 0.01%, 0,02% and 0.04% MMS (Methyl methanesulfonate). Plates were then incubated at 30°C for 48h.

### Gene conversion experiment

Strains were grown overnight in YPA+3%Raf medium, and diluted to OD₆₀₀ₙₘ=10⁻⁴. 100µl culture (corresponding to 100 cells) was plated separately on YPD and YP 2% galactose plate for 48h incubation at 30°C. Gene conversion efficiency was calculated by the number of colonies on YP-Gal plate divide by the corresponding number on YPD plate (Figure 2A, upper panel).

### Western blot

Cell lysates were extracted by the TCA method and 10min vigorous vortex at 4°C. After centrifuging, the pellets were resuspended in TCA-Laemmli loading buffer (120 mM Tris base, 3.5 % sodium dodecyl sulfate (SDS), 8 mM EDTA, 5 % β-mercaptoethanol, 1 mM PMSF, 15 % glycerol, 0.01 % bromophenol blue). Samples were incubated at 95°C for 10min. For immunoblotting, a polyclonal antibody anti-Rad51 (PA5-34905 ThermoFischer) was used at 1:3000.

### Immunofluoresence

A total of 20 OD600nm equivalent cells was fixed in 0.9% paraformaldehyde (Delta) and incubate at 30°C for 15min. Samples were centrifuged, washed twice with H2O, and resuspended in 1.25ml solution (0.1M EDTA-KO, 10mM DTT, pH=8.0) for 30 min incubation with a gentle agitation. After centrifugation, the pellets were resuspended in 1.25ml YPD-Sorbitol (1.2M) and 15µl zymolyase-100T. Cells were incubated at 30°C and their shape was checked every 5 min by microscopy (transmitted light). The reaction was stopped by adding 40ml YPD-Sorbitol when cells became round and didn't reflect anymore. The spheroplasts were washed twice with YPD-Sorbitol and finally resuspended in YPD without sorbitol. Cells were dropped on microscope slides (Polylabo, super-teflon slides) and and air dried for 3 min. Then, slides were put in methanol for 6 min and acetone for 30s at -20°C. After 3min air dry, the slides were incubated in 1×PBS with 1%BSA and 0.1%Triton X-100 for at least 20 min. Spots were covered by 1×PBS with 0.1% Triton X-100, anti-Rad51 antibody (PA5-34905 ThermoFischer, 1/500), and 1h incubation at 37 °C. After 3 washes in 1xPBS with 0.1% Triton X-100, the slides were dried and covered by Invitrogen goat anti-rabbit IgG(H+L) cross-adsorbed secondary antibody (Alexa fluor 568, catalog #A-11011, 1/100 in spheroplast suspension). After 1h incubation at 37°C and three times wash with 1xPBS,0.1% Triton X-100, nuclei were dyed with 1x DAPI (in 1×PBS) for 5min. After 2 washes with 1×PBS, 15µl antifading (DABCO, pH7.5) was added to each spot. Slides were covered with a cover slip and stored at 4°C in the dark.

### Microscopy

Yeast cells were grown in rich medium (YPD) overnight to early log-phase. To induce a single DSB at the I-Scel cut-site, cells were grown overnight in YPA Raf3% medium (yeast extracts, peptone, 3% Raffinose, 0.008 % Adenine HCl) and diluted to OD600nm=0.2 the next morning in the same medium. After 2h, galactose was added directly to the culture to reach a final concentration of 2%. Before microscopy, cells were rinsed with complete synthetic medium (2x final concentration pf CSM, MPBIO-101) and 3% Raffinose and placed on a 1.4% agarose patch for microscopy. For all fluorescent images, images were acquired in 3-dimensions with a z-step of 200nm: images shown are a maximum intensity projection of the z-stack images. Images were acquired on an inverted wide-field microscopy (Nikon TE2000) equipped with a 100x/1.4 NA immersion objective and a C-mos camara. Axenon arc lamp (Sutter Instrument Co. Lambda LS) and spectra X light engine lamp (Lumenor) were used to illuminate the samples. A dual of view micro-imager device, described in Guidi et al. (2015) allows the simultaneous measurement of 2-colors information on the same sensor.

### Time-lapse microscopy

For time-lapse microscopy, cells were grown overnight in complete synthetic medium (2x final concentration of CSM (yeast nitrogen base; MP Biomedicals) supplemented with 3% Raffinose. Cells were diluted to OD600nm=0.2 the next morning in the same medium. After 2h, galactose was added directly to the culture to reach a final concentration of 2%.

50µl of cells with OD=0.4 (diluted in the original filtered medium) were transferred to the microfluidic yeast plate (CellASIC Onix, using Y04C-02-5PK plates for haploid strains: and Y04D-02-5PK plates for diploid strains). The plates were driven by the Onix microfluidic perfusion system. Cells were loaded into the chamber in 15s = and set the flow pressure at 13.8kPa (2psi) during acquisition.

Time-lapse movies were acquired on a spinning-disk confocal microscopy equipped with a spinning-disk unit (Yokogawa CSU-X1), a Nikon Ti2000 statif, a 100x/1.4NA oil immersion objective and an EM CCD camera (ANDOR iXON DU-8850). The microscope is driven by MetaMorph software, and images were acquired every 2 or 5 minutes in 3-dimensions with z-steps spaces by 300 nm. The images shown in the figures are maximum intensity projections of the z-stacks.

### Quantification of the filaments' length

Filaments were cropped to 50x50 pixel size images and deconvolved using the Meinel algorithm in MetaMorph (eight iterations; sigma =0.8; frequency 3; MDS Analytical Technologies). The length quantifications were performed in 3-dimensions using a home-made macro: the segmentation is based on an automatic image thresholding using the Ostu method.

The intensity of the filaments was calculated after segmentation of the sum-projection images.

### Quantification through machine learning

2 ilastik (1.3.2 post1) projects were used to quantify the percentages of Rad51 structures, using a pixel and an object classification. At least 10 images were used for training. After segmentation on z-projection images, simple threshold (0.44, with 0.7 and 0.7 smooth) was applied on nuclei to count the total number of nucleus. Hysteresis threshold (core 0.7, final 0.85, smooth 0.5 and 0.5) was done on nucleus as well as Rad51 signal to classify Rad51 structures. Object features (shape, diameter, length of skeleton and intensity distributions) were used in both projects.

For intensity quantification, 3D filaments stacks (50x50 pixel) were input and possessed by a simple threshold (0.6, with 0.8 and 0.8 smooth). Size in pixels, mean intensity and total intensity were chosen for classification and output.

### RESULTS

### A fully functional internally tagged version of Rad51

So far, no fully functional version of fluorescent tagged Rad51 has been shown to be fully functional. After several adjustments and tests, inventors obtained endogenous tagged versions of Rad51, which allowed wild-type resistance to the damaging agent MMS when expressed as the only copy of *RAD51.* This was totally unexpected and in sharp contrast to the several attempts made in the art, and to the commonly used N or C-terminal tagged version (Figure 1, (*Lisby et al (2004), Waterman et al (2019))).* Of note, the peculiar site between Gly 54 and Gly 55 of the Rad51 of SEQ ID NO:1 is permissive to a large variety of protein tags : all the tested tags result in a cells with greatly improved MMS resistance compared to *rad51Δ* or N terminally tagged Rad51 protein, thereby signing a functional Rad51 protein,

Furthermore, internally tagged version of Rad51 is found as competent as the wild-type version to perform gene conversion following the induction of unique DSB in the presence of a homologous donor sequence located on a different chromosome (Figure 2A).

Also, western blotting shows that the sfGFP tag does not affect the expression levels of Rad51 even following the induction of an unrepairable DSB by the I-*Sce*l endonuclease (Figure 2B).

Similar results are observed with either sfGFP, yGFP, TagBFP2,Halo tag or even larger tagstag (not shown). These tags differ either in their activity and/or length. Together, these data show that Gly54-Gly55 site is particularly permissive to insert very different tags to obtain the internal tagged versions of Rad51 according to the invention which are functional for homologous recombination. This system can therefore be used to monitor Rad51 during HR events.

### Novel structures formed by Rad51 filaments.

Rad51 localization was monitored *in vivo* before and after induction of a unique un-repairable DSB in haploid cells using a fully functional tagged version of Rad51 (RAD51-sfGFP L16). In the absence of induced DSB spontaneous foci in 5 % of the cells are observed (Figure 3A) as previously shown using a N-terminal tagged version of Rad51 (*Lisby et al, 2004*). Similar foci are visible in cells 2 hours after I-Scel induction, together with brighter globular and elongated structures that were not reported for strains expressing either N- or C-terminal tagged Rad51 and have not been observed (not shown and (*Lisby et al (2004), Waterman et al (2019)).*

The percentage of cells showing Rad51 structures, quantified using machine-learning-based image analysis (Berg et al, 2019), increased over time after induction to reach 90 % after 6 hours (Figures 3 A and B). The proportion of elongated structures as well as their length also increased over time, some of them exceeding 1 µm in length at late time points akin the RecA filaments observed in bacteria.

Similar filaments were observed by immuno-fluorescence in untagged cells using an antibody raised against Rad51, after inducing an un-repairable DSB (not shown), therefore ruling out potential artefact owing to the presence of the tag. Filamentous structures were also observed in diploid cells in which the homologous chromosome provide a perfect donor sequence (Figure 4). In all situations, Rad51 structures were observed mainly in S and G2/M phases, as expected.

In diploid, the percentage of cells showing Rad51 structures peaks 4 hours after induction, a timing consistent with the disassembly of the nucleofilament once HR is achieved. Further it is observed that Rad51 filaments are the structures that associate the most with the donor sequence, whereas Rad51 foci or globular structures associates 3-fold less with the donor sequence, with no or low variation after DSB induction (not shown)

Therefore, Rad51 filaments observed here in living cells correspond to the functional structures performing the homology search and strand invasion during DSB repair.

Hence these data show that the internal tagged versions of Rad51 according to the invention allow a real time follow-up of cellular mechanisms during DSB repair, and therefore can be used a reporter of the activity homologous recombination DSB repair system in eukaryotic cells.

### Highlighting Rad51 Patterns and their dynamics in mutants for protein involved if DSB repair through homologous recombination

Rad51 filaments can adopt a variety of patterns (Figure 4) that can be categorized into 5 subclasses: rods, bent filaments, circles, branched structures with a single node and others (including more complex or multiple structures). Rad51 mainly forms rods and bent filaments at the early stage after DSB induction, while other shapes, are commonly observed after a 6h galactose induction in the wildtype strain, suggesting that the simple filaments convert into branched and circular structures over time (not shown). The same classes of structures were observed in the diploid strain, where the DSB is repaired, with notably a lower proportion of very complex structures (others). Rod, bent filaments, 1-node and circles filaments are then all functional structures.

Quantifying these patterns and structures make then possible to identify proteins or genes that interfere with HR of DSB. A *srs2*Δ strain show a close to wildtype distribution except that that rods and bent filaments are significantly longer in this strain (Figure 5), which is in agreement with the anti-recombinase activity of Srs2. Strikingly, *rad54Δ* strain has nearly no branched filaments but accumulates circular structures (>25% at 6 hours after DSB induction versus less than 10%, in WT and srs2Δ, Figure 5).

These results show that analyzing timing and shapes of nuclear-size structures formed by Rad51 filaments are tools to decipher HR repair mechanism within cells. As here for Srs2 and Rad54, possibly through modulation of Rad51 polymerization and capacity to invade dsDNA.

### Dynamics of Rad51 filament in vivo ; compaction/extension cycles.

The dynamics of Rad51 filament formation in living cells, was studied using time laps, taking one Z-stack of 21 images every 5 min, starting 90 min after I-Scel induction. It was observed that Rad51 first accumulates as a focus, whose intensity increases, before forming a more elongated structure or filament. Imaging cells, at 2 min time interval the median time between the appearance of the first focus and the formation of a structure larger than 300 nm is estimated to 22 min in wild-type cells (n=12). Consistently, this transition was faster in the absence of Srs2 or Rad54 (9 and 8.23 min respectively), indicating that both factors delay Rad51 filament elongation.

Once formed, it was observed also that Rad51 filaments are very dynamic, bending, changing orientation and shapes over time, switching from rod to bent filaments, to more complex structures, eventually disappearing in diploid cells, probably reflecting repair events. All classes of filament shapes observed in a population of cells have been observed in single movies. Therefore, the different classes of filament shapes reflect the dynamics behavior of Rad51 structures rather than cell to cell variability. Furthermore, abrupt changes in filament length, seemingly collapsing as bright foci, yet followed by a rapid re-extension are observed. These foci were not found to correspond to filament truncated in the Z axis or oriented perpendicularly to the XY plan. Also, the intensity of these foci was close to the total intensity of the extended filaments observed on the previous and following frames. Thus, these bright foci correspond to compacted filaments. This is reminiscent to the contraction events observed *in vitro* on Rad51 nucleoprotein filaments, which are hydrolyzing ATP, leading to shorter filaments associated with Rad51-ADP molecules that dissociate slowly from DNA (Hilario et al. (2009), Robertson et al (2009)).

Within over 26 hours of movies imaged at a 2 min rate, an average of one contraction event every 18 min is observed. These events also occur in the absence of Srs2 or Rad54 indicating that none of these factors are necessary for these events.

These cycles of extension/compaction/re-extension are thought to be implied in mechanism for homology search. Thus, their dynamics can be a data to consider to evaluate the Homologous recombination repair mechanism, easily assayed with internally tagged proteins as developed by the inventors.

### REFERENCES

Berg S., Kutra D., Kroeger T., Straehle C. N., Kausler B. X., Haubold C., Schiegg M., Ales J., Beier T., Rudy M., Eren K., Cervantes J. I., Xu B., Beuttenmueller F., Wolny A., Zhang C., Koethe U., Hamprecht F. A., Kreshuk A., ilastik: interactive machine learning for (bio)image analysis. Nat Methods. 16, 1226-1232 (2019).

Cloud V, Chan YL, Grubb J, Budke B, Bishop DK. Rad51 is an accessory factor for Dmc1-mediated joint molecule formation during meiosis. Science. 2012 Sep 7;337(6099):1222-5.

J. Hilario, I. Amitani, R. J. Baskin, S. C. Kowalczykowski, Direct imaging of human Rad51 nucleoprotein dynamics on individual DNA molecules. Proc. Natl. Acad. Sci. U.S.A. 106, 361-368 (2009).

Hofmarcher T, Brådvik G, Svedman C, Lindgren P, Jönsson B and Wilking N. Comparator Report on Cancer in Europe 2019 - Disease Burden, Costs and Access to Medicines. IHE Report 2019:7, IHE: Lund, Sweden.

Kobayashi W, Sekine S, Machida S, Kurumizaka H. Green fluorescent protein fused to the C terminus of RAD51 specifically interferes with secondary DNA binding by the RAD51-ssDNA complex. Genes Genet Syst. 2014;89(4):169-79.

Lisby M., Barlow J. H., Burgess R. C., Rothstein R., Choreography of the DNA Damage Response: Spatiotemporal Relationships among Checkpoint and Repair Proteins. Cell, 15 (2004). Olivier M, Charbonnel C, Amiard S, White Cl, Gallego ME. RAD51 and RTEL1 compensate telomere loss in the absence of telomerase. Nucleic Acids Res. 2018 Mar 16;46(5):2432-2445.

Needleman SB, Wunsch CD. A general method applicable to the search for similarities in the amino acid sequence of two proteins. J Mol Biol. 1970 Mar;48(3):443-53.

Reuter M, Zelensky A, Smal I, Meijering E, van Cappellen WA, de Gruiter HM, van Belle GJ, van Royen ME, Houtsmuller AB, Essers J, Kanaar R, Wyman C. BRCA2 diffuses as oligomeric clusters with RAD51 and changes mobility after DNA damage in live cells. J Cell Biol. 2014 Dec 8;207(5):599-613. Erratum in: J Cell Biol. 2015 Mar 16;208(6):857.

Robertson RB, Moses DN, Kwon Y, Chan P, Chi P, Klein H, Sung P, Greene EC. Structural transitions within human Rad51 nucleoprotein filaments. Proc Natl Acad Sci USA. 2009 Aug 4;106(31):12688-93.

Teixeira MT. Saccharomyces cerevisiae as a Model to Study Replicative Senescence Triggered by Telomere Shortening. Front Oncol. 2013 Apr 26;3:101.

Thorn K. Genetically encoded fluorescent tags. Mol Biol Cell. 2017 Apr 1;28(7):848-857. Waterman D. P., Zhou F., Li K., Lee C.-S., Tsabar M., Eapen V. V., Mazzella A., Haber J. E., Live cell monitoring of double strand breaks in S. cerevisiae. PLoS Genet. 15, e1008001 (2019). Waterman D. P., Zhou F., Li K., Lee C.-S., Tsabar M., Eapen V. V., Mazzella A., Haber J. E., Live cell monitoring of double strand breaks in S. cerevisiae;bioRxiv 265611

Yu DS, Sonoda E, Takeda S, Huang CL, Pellegrini L, Blundell TL, Venkitaraman AR. Dynamic control of Rad51 recombinase by self-association and interaction with BRCA2. Mol Cell. 2003 Oct;12(4):1029-41.

## Claims

1. A fully functional tagged Rad51 protein **characterized in that** it comprises a tag between residues 54 and 55 of Rad 51 protein of SEQ ID NO:1 or between corresponding residues of any budding yeast ortholog thereof.

2. The fully functional tagged Rad51 protein **characterized in that** the tag is a protein tag.

3. The fully functional tagged Rad51 protein according to claim 1 or 2 wherein said tag is a fluorescent protein tag selected from mTagBFP2, TagBFP2, mTurquoise2, mCerulean3, EGFP, mEOS3.2, mWasabi, Superfolder GFP (sfGFP), mNeonGreen, mClover3, Venus, Citrine, mKOκ, tdTomato, TagRFP-T, mRuby3, mScarlet, FusionRed, mStable, mKate2, mMaroon1, mCardinal, T-Sapphire, mCyRFP1, LSSmOrange, mBeRFP, CreiLOV, UnaG, miRFP670, TDsmURFP, iRFP670, mIFP, iFP2.0, iRFP720 EBFP, ECFP, yEGFP, YFP, mHoneydew, mOrange, mTangenine, mStrawberry, mCherry, MGrape1, mRaspberry, mGrape2, mPlum or Halo.

4. The fully functional tagged Rad51 protein according to any one of claims 2 or 3 wherein said protein tag is flanked by peptide linkers, each of at least 11 amino acids long.

5. The fully functional tagged Rad51 protein according to the preceding claim wherein said peptide linkers comprise at least 1, preferably at least 2, even more preferably at least 3 positively charged amino acid residues at 2 to 4 positions from anchoring residues of the protein tag.

6. The fully functional tagged Rad51 protein according to claims 4 or 5 wherein the linker in Nter of the protein tag is of sequence SEQ ID 9 and the linker in Cter of the protein tag is of sequence SEQ ID 10 or wherein the linker in Nter of the protein tag is of sequence SEQ ID 11 and the linker in Cter of the protein tag is of sequence SEQ ID 12.

7. A cell comprising a nucleic acid encoding for a fully functional tagged Rad51 protein of a budding yeast **characterized in that** it comprises a tag between residue 54 and 55 of Rad51 protein of SEQ ID 1 or between corresponding residues of any budding yeast ortholog thereof.

8. A method for screening for a candidate compound capable of interfering with homologous recombination of DNA upon double strand break repair and/or in the alternative lengthening of telomere recombination pathway involving Rad51, comprising:
- contacting with said candidate compound a cell comprising a nucleic acid encoding for a eukaryotic fully functional tagged Rad51 protein **characterized in that** it comprises a protein fluorescent tag between residues 54 and 55 of Rad51 protein of SEQ ID NO 1 or between corresponding residues of any budding yeast ortholog thereof,
- analysing in said cell at least one type of structure comprising the tagged Rad51 without and upon double strand break induction, and/or with or without at least one short telomere,
- selecting a candidate compound which induces, when compared to at least one control condition, an alteration in said at least one type of structure comprising the tagged Rad51 protein, as a candidate compound capable of interfering with homologous recombination of DNA upon double strand break repair and/or in the alternative lengthening of telomere recombination pathway involving Rad51.

9. The method according to claim 8, wherein the at least one type of structure comprises a filament, foci, globular structures comprising said tagged Rad51 protein.

10. The method according to any of claims 8 and 9, wherein the alteration comprises a deviation, when compared to the at least one control condition, in the number, the shape, the brightness and/or the dynamics of said at least one type of structure.

11. The method according to any of claims 8-10, wherein the alteration comprises an increase of the rate of the compaction/extension cycles of Rad51 filaments, with an average contraction event occurring in a time laps of more than 20 min.

12. The method according to any of claims 8-11, wherein the alteration comprises a decrease of the length of the filament that is formed upon double strand break induction.

13. A computer implemented method of analysing homologous recombination of DNA upon double strand break repair mechanism and/or the alternative lengthening of telomere recombination pathway involving Rad51, comprising:
- detecting, in at least one image of at least one cell comprising a nucleic acid encoding for a fully functional tagged Rad51 protein of a budding yeast **characterized in that** it comprises a fluorescent protein tag between residue 54 and 55 of Rad51 protein of SEQ ID 1 or between corresponding residues of any budding yeast ortholog, at least one type of structure comprising said Rad51 protein tagged with a fluorescent protein between residues 54 and 55,
- determining the number, the shape, the brightness and/or the dynamics of said at least one type of structure,
wherein said number, shape, brightness and/or the dynamics of said at least one type of structure characterizes, in said at least one cell, homologous recombination of DNA upon double strand break repair mechanism and/or the alternative lengthening of telomere recombination pathway involving Rad51.

14. A method for screening for a candidate compound capable of sensitizing a cancer cell to a chemotherapeutic agent and/or exposure to radiations comprising:
- contacting with said candidate compound a cell comprising a nucleic acid encoding for a fully functional tagged Rad51 protein **characterized in that** it comprises a protein fluorescent tag between residue 54 and 55 of Rad51 protein of SEQ ID NO: 1 or between corresponding residues of any budding yeast ortholog thereof,
- analysing in said cell at least one type of Rad51 structure without and upon double strand break induction, and/or with or without at least one short telomere.
- selecting a candidate compound which induces an alteration in said at least one type of structure comprising the tagged Rad51 protein as a candidate compound capable of interfering with homologous recombination of DNA upon double strand break repair and/or in the alternative lengthening of telomere recombination pathway involving Rad51,
- contacting at least one cancer cell with the compound selected in preceding step before or concomitantly to a treatment with a chemotherapeutic agent and/or ionising radiation,
if an increase in death rate of cancer cell is reported in regard to control cancer cells not contacted with the selected compound and submitted to said treatment with a chemotherapeutic agent or ionising radiation, then selecting said compound as a compound capable of sensitizing a cancer cell to chemotherapeutic agent and/or radiotherapy.

15. The *in vitro* or *in cellulo* use of a fully functional tagged Rad51 protein **characterized in that** it comprises a tag between residues 54 and 55 of Rad 51 protein of SEQ ID 1 or between corresponding residues of any budding yeast ortholog thereof, as a reporter of DNA homologous recombination upon double strand break repair mechanism and/or of the alternative lengthening of telomere recombination mechanism involving Rad51.
